# EUROPÄISCHE PATENTSCHRIFT

(11) **EP 3 321 370 B1**
(45) Veröffentlichungstag und Bekanntmachung des Hinweises auf die Patenterteilung: **02.09.2020**
(21) Anmeldenummer: 16198489.3
(22) Anmeldetag: 11.11.2016
(51) Int. Cl.: C12Q 1/14, C12Q 1/04

(54) **ERFINDUNG BETREFFEND DIE STEIGERUNG DER BETA-HÄMOLYSE VON CDC-TOXIN PRODUZIERENDEN MIKROORGANISMEN**
INVENTION RELATING TO INCREASING THE BETA HEMOLYSIS OF MICROORGANISMS PRODUCING CDC TOXIN
INVENTION CONCERNANT L'AUGMENTATION DE L'HÉMOLYSE BETA DES MICROORGANISMES PRODUISANT DES TOXINES CDC

(43) Veröffentlichungstag der Anmeldung: 16.05.2018
(73) Patentinhaber: Justus-Liebig-Universität Gießen, 35390 Gießen (DE)
(72) Erfinder: Chakraborty, Trinad Prof., 35394 Giessen (DE); Pillich, Helena Dr., 35396 Giessen (DE)
(74) Vertreter: Stumpf, Peter

(56) Entgegenhaltungen:
- LING LI ET AL: "Characterization of Plp, a phosphatidylcholine-specific phospholipase and hemolysin of Vibrio anguillarum", BMC MICROBIOLOGY, BIOMED CENTRAL LTD, GB, Bd. 13, Nr. 1, 27. November 2013 (2013-11-27), Seite 271, XP021169253, ISSN: 1471-2180, DOI: 10.1186/1471-2180-13-271
- K CHRISTIE ET AL: "A NOTE ON A LYTIC PHENOMENON SHOWN BY GROUP B STREPTOCOCCI", AUSTRALIAN JOURNAL OF EXPERIMENTAL BIOLOGY AND MEDICAL SCIENCE, Bd. 22, Nr. 3, 1. September 1944 (1944-09-01), Seiten 197-200, XP055346878, AU ISSN: 0004-945X, DOI: 10.1038/icb.1944.26
- R C Mckellar: "Use of the CAMP Test for Identification of Listeria monocytogenes", APPLIED AND ENVIRONMENTAL MICROBIOLOGY, Bd. 60, Nr. 12 1. Dezember 1994 (1994-12-01), Seiten 4219-4225, XP055346934, Gefunden im Internet: URL:http://aem.asm.org/content/60/12/4219. full.pdf [gefunden am 2017-02-16]
- KIMBERLY J. RAMSEY ET AL: "Reclassification of the Listeria-CAMP Test Strain ATCC 49444 Staphylococcus aureus as Staphylococcus pseudintermedius", JOURNAL OF FOOD PROTECTION, Bd. 73, Nr. 8, 1. August 2010 (2010-08-01) , Seiten 1525-1528, XP055346886, US ISSN: 0362-028X, DOI: 10.4315/0362-028X-73.8.1525
- H B Ratner ET AL: "Evaluation of spot CAMP test for identification of group B streptococci", Journal of Clinical Microbiology, Bd. 24, Nr. 2 1. August 1986 (1986-08-01), Seiten 296-297, XP055346935, UNITED STATES Gefunden im Internet: URL:https://www.ncbi.nlm.nih.gov/pmc/artic les/PMC268893/pdf/jcm00098-0142.pdf [gefunden am 2017-02-16]

## Beschreibung

Die Erfindung betrifft die Verwendung eines Differentialnährbodens umfassend Heparin zur Diagnostik von CDC-Toxin produzierenden Bakterien, wobei eine biologische Probe auf dem Differentialnährboden ausgebracht, kultiviert und auf das Vorhandensein von β-Hämolyse ausgewertet wird. Dies ermöglicht eine verbesserte, zuverlässige, sichere, schnelle und kostengünstig durchführbare Diagnostik für CDC-Toxin produzierende Mikroorganismen. Heparin wird einem Differentialnährboden zugefügt. Heparin wird in einem Hämolysintitertest oder einem Cytotoxizitätstest zugefügt. Der verbesserte Differentialnährboden, sowie beide Tests führen zu einer deutlichen Steigerung der β-Hämolyse und damit zu einer verbesserten Diagnostik für CDC-Toxin produzierende Mikroorganismen.

Zu den CDC-Toxin produzierenden Mikroorganismen gehören mehr als 40 grampositive Bakterien wie *Arcanobacterium pyogenes, Bacillus anthracis, Bacillus cereus, Bacillus sphaericus, Bacillus thuringiensis, Brevibacillus laterosporus, Brevibacillus brevis, Clostridium bifermentans, Clostridium botulinum, Clostridium butyricum, Clostridium chauvoei, Clostridium histolyticum, Clostridium novyi, Clostridium perfringens, Clostridium septicum, Clostridium sordelli, Clostridium tetani, Gardnerella vaginalis, Listeria ivanovii, Listeria monocytogenes, Listeria seeligeri, Paenibacillus alvei, Streptococcus canis, Streptococcus dysgalactiae, Streptococcus intermedius, Streptococcus mitis, Streptococcus pneumoniae, Streptococcus pyogenes, Streptococcus suis* und *Streptococcus pseudopneumoniae.*

Von diesen CDC-Toxin produzierenden Mikroorganismen sind *Streptococcus pneumoniae, Clostridium perfringens, Listeria monocytogenes, Bacillus cereus, Bacillus anthracis, Streptococcus pyogenes* und *Clostridium tetani* für Menschen und Tiere pathogen. Für diese Bakterien ist eine sichere Diagnostik daher besonders wichtig.

Die CDC-Toxin produzierenden Mikroorganismen weisen mindestens ein CDC-Toxin auf. CDC-Toxine sind Proteine mit einer bestimmten Aminosäuresequenz. Sie werden auch als Cholesterol-abhängige Zytolysine oder Thiol-aktivierte Zytolysine bezeichnet (englisch cholesterol-dependent cytolysin, CDC). Beispiele sind Pneumolysin aus *Streptococcus pneumoniae,* Perfringolysin O aus *Clostridium perfringens,* Listeriolysin O aus *Listeria monocytogenes,* Ivanolysin aus *Listeria ivanovii,* Seeligerolysin aus *Listeria seeligeri,* Cereolysin aus *Bacillus cereus,* Alveolysin aus *Bacillus alvei,* Streptolysin aus *Streptococcus pyogenes* oder Tetanolysin aus *Clostridium tetani.*

Beispielsweise wird hier die Aminosäuresequenz des CDC-Toxins von Listeriolysin O (abgekürzt als LLO) von *Listeria monocytogenes* Stamm EGDe (Seq.ID1 und Figur 12). Diese Sequenz ist im Stand der Technik bereits bekannt und offenbart in Köster, van Pee, Hudel, Leustik, Rhinow, Kühlbrandt, Chakraborty, Yildiz. Crystal structure of listeriolysin O reveals molecular details of oligomerization and pore formation. Nat Commun. 2014 Apr 22;5:3690. doi: 10.1038/ncomms4690.

CDC-Toxine gehören zu den porenbildenden Toxinen (englisch pore forming toxin, PFT). Mit ihnen bilden die Bakterien in den Zellmembranen von Wirtszellen (von Mensch oder Tier) Poren von bis zu 250 Å (25 nm) Durchmesser, sodass diese Zellmembranen perforiert werden. Als Folge läuft das Zytosol aus und es kommt zum Absterben der betroffenen Zellen. Die CDC-Toxine haben somit eine toxische Wirkung auf die Wirtszellen. Durch den entstandenen Schaden für den Wirt werden die meisten porenbildenden Toxine zu den Virulenzfaktoren gezählt.
Die Aminosäuresequenz der verschiedenen CDC-Toxine weist eine Homologie von mindestens 40% bis zu 70% auf. Daher gehören im Rahmen der vorliegenden Erfindung auch die Bakterien zu den CDC-Toxin produzierenden Bakterien, die Proteine mit einer mindestens 40%igen Aminosäuresequenzidentität mit Seq.ID 1 aufweisen. Damit sind erfindungsgemäß auch jene Bakterien umfasst, die in der gängigen Nomenklatur noch nicht zweifelsfrei als CDC-Toxin produzierende Bakterien identifiziert sind.

Zur Diagnostik von pathogenen Bakterien nutzt der Fachmann die hämolytische Eigenschaft der CDC-Toxin produzierenden Bakterien. Hämolytische Eigenschaft oder Hämolyse bezeichnet dabei die Fähigkeit dieser Bakterien, Erythrozyten zu zersetzen. Diagnostisch erfolgt dies mittels Ausstrich auf Platten mit Blutagar als Differentialnährboden. Der Blutagar enthält Erythrozyten z.B. aus Blut eines Wirbeltieres, beispielsweise Schaf. Der Blutagar weist eine rote Farbe aufgrund des in den Erythrozyten enthaltenen Hämoglobins auf. Das Vorhandensein von Bakterien mit hämolytischer Eigenschaft zeigt sich in der Bildung einer Zone auf der Platte, in der die rote Farbe fehlt (auch "Hof" genannt). Der Fachmann unterscheidet allgemein zwischen einer α-, β- und γ-Hämolyse:
- **α-Hämolyse,** auch Vergrünung genannt. Hier verursachen die Bakterien eine grünliche Zone auf dem Blutagar, die nicht durch echte Hämolyse hervorgerufen wird, sondern auf einer Entfärbung und einem Kaliumverlust der Erythrozyten basiert. Die grüne Färbung erfolgt durch Reduktion des Hämoglobins zu Biliverdin.
- **β-Hämolyse,** hier bilden die Bakterien durch die porenbildenden Toxine eine klare Hämolysezone, in der das Hämoglobin vollständig abgebaut und die Erythrozyten vollständig hämolysiert werden.
- **γ-Hämolyse,** hier zeigen die Bakterien keinerlei Hämolyseverhalten.

Bakterielle porenbildende Toxine, die die β-Hämolyse auslösen, werden in verschiedene Gruppen eingeteilt, die sich hinsichtlich des Aufbaus und des Wirkmechanismus unterscheiden:
- α-porenbildende Toxine, z. B. Cytolysin A
- β-porenbildende Toxine, z. B. α-Hämolysin, PV-Leukozidin
- Cholesterol-abhängige Zytolysine (= CDC-Toxine)
   z. B. Listeriolysin O (LLO), Pneumolysin
- Kleine porenbildende Toxine, z. B. Gramicidin A

Die Erfindung betrifft die Verwendung eines Differentialnährbodens umfassend Heparin zur Diagnostik von CDC-Toxin produzierenden Bakterien wobei eine biologische Probe auf dem Differentialnährboden ausgebracht, kultiviert und auf das Vorhandensein von β-Hämolyse ausgewertet wird. In der aktuellen Analytik ist dabei die Detektion von Listerien besonders wichtig, deshalb wurden viele Ausführungsbeispiele mit diesem Bakterium durchgeführt.

Bakterien der Gattung *Listeria (L.)* sind grampositive, bewegliche, nichtsporenbildende, katalasepositive und fakultativ anaerobe Stäbchen. *L. monocytogenes* ist eine bedeutende humanpathogene Spezies, die sich in 13 Serovare subdifferenzieren (Serotypie) lässt, von denen die Serovare 4b, 1/2a und 1/2b besonders mit Erkrankungen des Menschen assoziiert sind. *L. monocytogenes* ist ein fakultativ pathogener Erreger (Opportunist), der auch bei Tieren vorkommt, jedoch auch außerhalb des tierischen Organismus überleben und sich vermehren kann. Im infizierten Tier oder Menschen kann sich *L. monocytogenes* intrazellulär vermehren und direkt von einer Wirtszelle in die Nachbarzelle vordringen, ohne dass sie dabei in das extrazelluläre Milieu übertreten müssen. Medizinisch bedeutsam ist vor allem das Eindringen und Vermehren in Epithelzellen, wodurch Listerien aktiv anatomische Barrieren (Blut-Hirn-, Darm- und Plazenta-Schranke) überwinden. *L. monocytogenes* sowie die übrigen Listeria-Spezies sind weltweit verbreitet. Sie kommen ubiquitär in der Umwelt vor, insbesondere in der Erde und auf Pflanzen, in Abwässern und allgemein im landwirtschaftlichen Bereich. Die Bakterien werden häufig im Tierfutter (besonders in verdorbener Silage), im Kot von Tieren und sogar im Stuhl gesunder Menschen nachgewiesen.

Die Listeriose ist eine durch Bakterien der Gattung *Listeria* verursachte lebensmittelbedingte Infektionskrankheit. Listerien werden auf einer Vielzahl tierischer Lebensmittel wie Geflügel, Fleisch, Fleischprodukte (z.B. Wurst), Fisch, Fischprodukte (hauptsächlich Räucherfisch), Milch und Milchprodukte (insbesondere Käse) gefunden. Zudem werden Listerien auch häufig auf pflanzlichen Lebensmitteln (z.B. vorgeschnittenen Salaten) gefunden. Eine Kontamination dieser Lebensmittel mit Listerien kann dabei an verschiedenen Stellen der Gewinnung und Bearbeitung erfolgen, z.B. beim Melken, beim Schlachten oder durch eine Kontamination über die Umwelt.

Zudem führt die Verarbeitung und Behandlung der kontaminierten Rohstoffe nicht immer zu einer vollständigen Abtötung der Bakterien, z.B. bei Rohmilchweichkäse, Rohwurst oder Hackfleisch. Neben einer Kontamination des Ausgangsmaterials können Listerien aber auch in lebensmittelverarbeitenden Betrieben gefunden werden. Ihre Anwesenheit kann zu einer Rekontamination auch derjenigen Lebensmittel führen, die einem Erhitzungsprozess oder einem anderen, Listerienabtötenden Verfahren unterzogen wurden.

Die Listeriose kommt beim Menschen vor allem bei Schwangeren und deren ungeborenen Kindern sowie bei Neugeborenen, bei alten Menschen und bei Menschen mit einer abgeschwächten Immunabwehr vor. In der Tierwelt sind vor allem Wiederkäuer (Ziegen, Schafe und Rinder) betroffen, selten auch Vögel, Pferde, Schweine oder Raubtiere. Die Verbreitung bei Mensch und Tier erfolgt insbesondere durch verdorbene und verschmutzte Lebens- beziehungsweise Futtermittel, weshalb die Listeriose keine echte Zoonose (eine von Tieren auf Menschen übertragbare Krankheit), sondern vielmehr eine Fäulnis- (Sapronose) oder Erdkeiminfektion (Geonose) ist. Die Listeriose ist in Deutschland seit 2001 sowohl beim Menschen als auch bei Tieren eine meldepflichtige Krankheit gemäß Infektionsschutzgesetz und Verordnung über meldepflichtige Tierkrankheiten. Schwangerschaftassoziierte Listeriosen betreffen 10% aller Listeriose-Meldefälle, wobei in dieser Berechnung sowohl die Mutter als auch das Neugeborene einfließen. Die nicht-schwangerschaftsassoziierten Listeriosen betreffen vor allem die Altersgruppen ab 50 Jahre. Viele der Betroffenen sind durch eine Grunderkrankung immunsupprimiert (z.B. Tumoren, Transplantation, Patienten unter Cortison-Therapie), wobei Männer mit 65% häufiger betroffen sind als Frauen.

Die Krankheit ist bei 30% aller Erkrankten mit einer Meningitis und bei 30% mit einer Sepsis assoziiert. Die Listeriose gehört zu den meldepflichtigen Erkrankungen mit der höchsten Letalität, wobei die Letalität im Durchschnitt bei 30% liegt. Das klinische Bild der Listeriose ist sehr variabel und hängt vor allem vom befallenen Organsystem ab. Daher ist die Erkrankung klinisch nicht sicher festzustellen, sodass eine adäquate Behandlung mit wirksamen Antibiotika häufig zu spät erfolgt. Grundsätzlich kann im Verlauf einer Listeriose jedes Organ befallen werden und es können verschiedene lokalisierte, eitrige Infektionen wie z.B. Arthritis, Endokarditis oder Konjunktivitis auftreten.

Eine Listeriose äußert sich auch als lokale Wundinfektion sowie als Entzündung der Binde- und Hornhaut (= Keratokonjunktivitis). Bei Lebensmittelinfektionen treten beim Menschen im Regelfall zunächst Durchfall und Bauchschmerzen auf. Am häufigsten entwickeln sich im weiteren Verlauf bei Mensch und Tier infolge einer Entzündung des Gehirns und der Hirnhäute zentralnervöse Störungen wie Lähmungen, Zittern, Körperfehlstellungen und Benommenheit. Bei Schwangeren und trächtigen Tieren kann es zu Fehlgeburten, zum Absterben des Fötus oder zu einer schweren Neugeborenensepsis kommen.

Die Diagnostik ist sehr schwer, da das Krankheitsbild divers ist und je nach Ausbildung als Meningitis, Sepsis oder Gastroenteritis auch andere bakterielle Infektionen ursächlich sein können. Die Labordiagnostik in der Humanmedizin basiert auf dem Erregernachweis aus Blut, Liquor, Eiter, Vaginalsekret, Lochien, Stuhl, Mekonium oder Biopsie- bzw. Autopsiematerial. Die Art des kulturellen Nachweises richtet sich nach der zu erwartenden Begleitflora in der Probe (direkter kultureller Nachweis bzw. Anzucht des Erregers nach Anreicherung). Listerien verursachen zwar febrile Gastroenteritiden, werden andererseits aber auch bei bis zu 5% gesunder Personen im Stuhl gefunden.

Eine bekannte Nachweismethode von *L. monocytogenes* in biologischen Proben erfolgt auf Basis von β-Hämolyse in Blutagar. Eine typische Zusammensetzung von Blutagar ist:

| **Nährsubstrat (Herzextrakt und Peptone)** | **20,0 g/Liter** |
|---|---|
| **Natriumchlorid** | **5,0 g/Liter** |
| **Agar-Agar** | **15,0 g/Liter** |
| **Blut** | **50-80 ml** |

Die *L. monocytogenes* Stämme werden auf Schafblutagar gezüchtet, wobei die Morphologie ähnlich wie bei Gruppe-B-Streptokokken ist. Um eine Differenzierung gegenüber anderen *Listeria-Spezies* zu erreichen, wird ein CAMP-Test (benannt nach **C**hristie-**A**tkins-**M**unch-**P**etersen) durchgeführt, bei dem Listerien einer Probe in Nachbarschaft von β-Lysin-produzierenden *Staphylococcus aureus* auf eine Blutagarplatte ausgestrichen und bebrütet werden.

Bei Vorliegen von *L. monocytogenes* wird Listeriolysin O (LLO) produziert, welches in den Agar diffundiert. Die LLO-bedingte β-Hämolyse wird durch das ebenfalls in den Agar diffundierende β-Lysin von S. *aureus* verstärkt.

Es sind weitere biochemische Untersuchungen wie z.B. MALDI-TOF-Massenspektrometer (**M**atrix-**A**ssistierte **L**aser-**D**esorption-**I**onisierung mit Flugzeitanalyse, engl. *time of flight),* ELISA (**E**nzyme **L**inked **I**mmunosorbent **A**ssay) oder molekularbiologische Untersuchungen wie z.B. PCR (Polymerase-Kettenreaktion, engl. **p**olymerase **c**hain **r**eaction) bekannt. Allerdings sind diese Methoden nur in einem Spezial- oder Referenzlabor durchführbar und mit hohen Kosten verbunden.

In der Lebensmittelanalytik gibt es mikrobiologische Nachweisverfahren basierend auf Anreicherung in einem speziellen Nährmedium. Diese Nachweisverfahren liefern erst nach 5 - 7 Tagen ein Ergebnis und dauern damit verhältnismäßig lange. Im Stand der Technik haben Fraser und Sperber die sogenannte Fraser-Bouillon entwickelt um die Anwesenheit von Listerien in Proben bestimmen zu können. Das Nachweisprinzip besteht dabei darin, dass alle Listeria spp. Äskulin hydrolysieren. In der Fraser-Bouillon reagieren Eisenionen mit den Produkten der Äskulinhydrolyse zu stabilen Komplexen, erkennbar an einem schwarzen Präzipitat. Nach dem Bebrüten werden Anreicherungskulturen, die sich schwärzen, als Listerienverdächtig bezeichnet. Die Schwärzung vollzieht sich besser bei 35°C als bei 30°C und ist nach 24-48 h komplett abgeschlossen. Allerdings ist dieses Medium nicht spezifisch, denn auch Anreicherungskulturen, die keine Listerien enthalten, färben sich schwarz.

Im Stand der Technik ist auch Fraser-Bouillon mit dem selektiven Agens Acriflavin bekannt. Acriflavin beeinträchtigt das Wachstum von *L. monocytogenes,* nicht aber das von *L. innocua* (nicht pathogen). Damit ist also eine indirekte Differenzialdiagnose möglich, die jedoch nicht spezifisch für CDC-Toxin enthaltende Bakterien ist. Weiterhin bekannt ist Fraser-Bouillon mit dem Zusatz von Nalixidin oder das Wachstum auf Selektivnährmedium (PALCAM- bzw. Oxford-Agar/ALOA). ALOA ist ein chromogenes Selektiv- und Differenzierungsmedium nach Ottaviani und Agosti.

Auch im PALCAM-Agar (basiert auf der Rezeptur von van Netten et al.) sind Äskulin und Eisen enthalten, so dass nach 24 h Inkubation ca. 1 mm große, graugrüne Kolonien entstehen, die regelmäßig nach 48 h in den Zentren eingesunken und von schwarzen Höfen umgeben sind.

Die bisher bekannten Ergebnisse zeigen eine Abhängigkeit der tatsächlichen Sensitivität der festen selektiven Nährmedien von der Art der untersuchten Lebensmittelprobe. In vergleichenden Studien konnte kein Nährbodentyp in jedem Fall Listerien aus den Proben identifizieren. Daraufhin wurde die Verwendung zweier Nährmedientypen vorgeschlagen. Das entscheidende Problem in der Diagnostik mit Nährmedien ist, dass die biologischen Proben wie Lebensmittel, Blut, Serum, Liquor, Eiter, Vaginalsekret, Lochien, Stuhl, Mekonium oder Biopsie- bzw. Autopsiematerial häufig Mischkontaminationen enthalten, in denen neben *L. monocytogenes* auch *L. innocua* enthalten ist.

Es besteht der Bedarf an einer verbesserten Diagnostik von CDC-Toxin produzierenden Mikroorganismen insbesondere Listerien, die schnell und zuverlässig gute Resultate liefert. Diese Diagnostik ist nicht nur wichtig, um bei Patienten, die eine lebensmittelbedingte *L. monocytogenes*-Infektion aufweisen, schnell und zuverlässig zu erkennen sondern auch für Krebspatienten, denn in den letzten Jahren werden *L. monocytogenes-*Stämme, die nicht-hämolytisches LLO sekretieren, für die Krebs-Immuntherapie eingesetzt. Hierbei werden *L. monocytogenes*-LLO-Stämme als Vektoren eingesetzt, um die angeborene und adaptive Immunantwort zu induzieren und so eine anti-Tumor Antwort zu erhöhen (Advaxis, Inc). Um bei den so therapierten Patienten keine Listeriose auslösen, ist es von größter Wichtigkeit, während des Verlaufs der Therapie *L. monocytogenes* auf die Hämolysefähigkeit zu untersuchen.

### Aufgabe

Aufgabe der vorliegenden Erfindung ist es, eine verbesserte Detektion von CDC-Toxin produzierenden Mikroorganismen in einer biologischen Probe zur zuverlässigen, sicheren, schnellen und kostengünstigen Diagnostik bereitzustellen.

### Lösung der Aufgabe

Diese Aufgabe wird durch ein Differentialnährboden umfassend Heparin zur Steigerung der β-Hämolyse von mindestens einem CDC-Toxin produzierenden Mikroorganismus gelöst.

Es werden zwei alternative Nachweisverfahren zur Detektion von CDC-Toxin produzierenden Mikroorganismen in biologischen Proben bereitgestellt. Eine biologische Probe ist z.B. eine Blut-, Serum- oder Stuhlprobe, eine Probe aus einem Gewebe oder Organ, ein Bioptat, ein Kosmetikum, aber auch eine Lebensmittelprobe, eine Rückstellprobe enthaltend ein verarbeitetes Lebensmittel oder eine Probe aus der Umwelt, wie Boden- oder Wasserproben. Die biologische Probe kann also fest oder flüssig sein. Der Fachmann kennt Methoden im Stand der Technik, um die feste biologische Probe zu verflüssigen. Zum Beispiel wird die feste Probe in sterile Lösung gegeben (z.B. NaCl) und mittels Homogenisierung zerkleinert (z.B. mit FastPrep® Homogenizer), so dass sich die Bakterien in Lösung befinden.

Überraschenderweise wurde in eigenen wissenschaftlichen Arbeiten gefunden, dass Heparin die β-Hämolyse von CDC-Toxin produzierenden Mikroorganismen sehr stark steigert. Dies kann besonders in einem mit Heparin versetzten Differentialnährboden z.B. Blutagar beobachtet werden. Daraufhin ist es überraschenderweise gelungen, mittels Heparin die gängigen Verfahren Hämolysintitertest und Cytotoxizitätstest so zu verbessern, dass diese zur zuverlässigen, sicheren und wesentlich schnelleren Diagnostik von CDC-Toxin produzierenden Mikroorganismen eingesetzt werden können.

Durch die Zugabe von Heparin zum Nährmedium z.B. zu Blutagar liegt ein verbesserter Differentialnährboden vor, auf dem die CDC-Toxin produzierenden Mikroorganismen aus einer biologischen Probe oder einer Referenzprobe eine deutliche und gut sichtbare β-Hämolyse zeigen.

Die Detektion von CDC-Toxin produzierenden Mikroorganismen in einer biologischen Probe erfolgt mittels eines verbesserten Hämolysintitertests oder verbesserten Cytotoxizitätstests.

Bei beiden Tests wurde überraschenderweise die vorteilhafte Wirkung von Heparin als Mittel zur Steigerung der β-Hämolyse bestätigt, indem die Zelllyse kernloser versus kernhaltiger sowie adhärenter (anhaftender) versus nicht adhärenter (nicht anhaftender) Zellen ermittelt wird. Der verbesserte Differentialnährboden, sowie der verbesserte Hämolysintitertest und verbesserte Cytotoxizitätstest ermöglichen eine zuverlässige und schnelle Detektion von *L. monocytogenes* sowie eine Unterscheidung von anderen Listerien-Arten und anderen Mikroorganismen die keine CDC-Toxine produzieren. Dieser Differentialnährboden, der Hämolysintitertest und der Cytotoxizitätstest haben den Vorteil, dass sie die β-Hämolyse von *L. monocytogenes* verstärken, während nicht pathogene Listerienarten und andere Mikroorganismen nicht hämolytisch bleiben, da sie keine CDC-Toxine aufweisen.

Heparin ist ein Glykosaminglykan, bestehend aus einer variablen Anzahl von Aminozuckern mit einer molaren Masse zwischen 4.000 und 40.000. Heparine sind variabel verestert und umfassen abwechselnde Folgen von D-Gukosamin und entweder einer D-Glukuronsäure oder L-Iduronsäure. Viele Monomereinheiten enthalten an Sauerstoff- und Stickstoffatome gebundene Sulfatgruppen. Ab einer Kettenlänge von 5 Monosachariden wirken Heparine gerinnungshemmend.

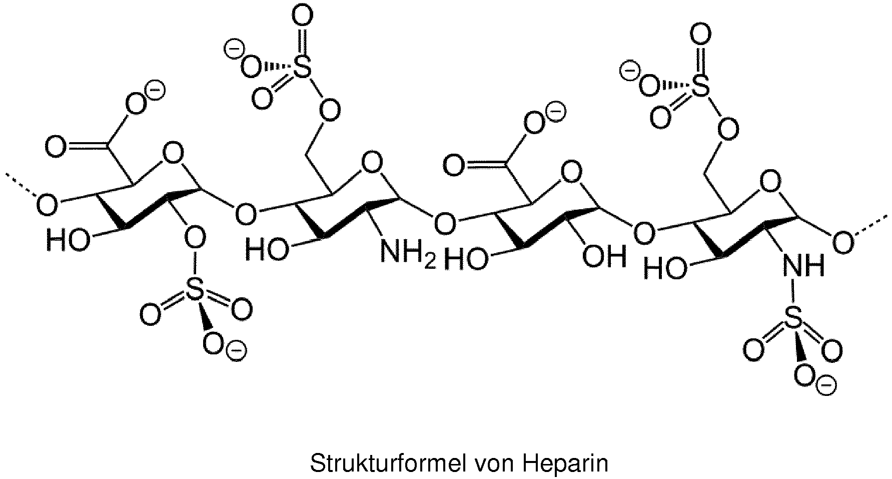

In der Regel wird Heparin aus der Darmschleimhaut von Schweinen gewonnen und gereinigt. Allerdings ist auch eine synthetische Herstellung möglich. Weiterhin können Heparin-Oligosaccharide oder modifizierte Heparine (z.B. Gallensäure-acyliertes Heparinderivat) verwendet werden, die nach einem dem Fachmann bekannten Verfahren synthetisch hergestellt wurden.

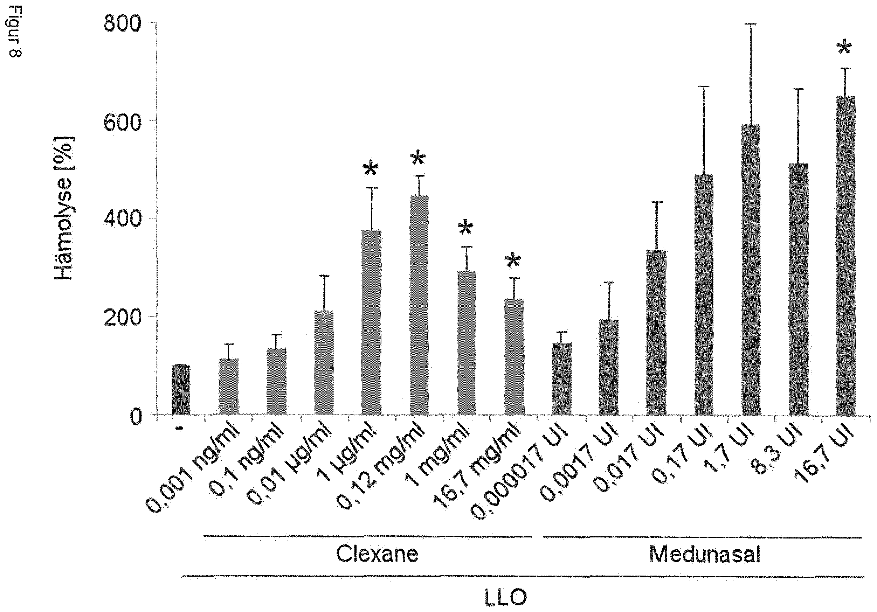

Überraschenderweise bewirkt Heparin auch eine Aktivitätssteigerung der Cholesterol-bindenden Zytolysine (auch Thiol-aktivierte Zytolysine) wie Listeriolysin O (LLO) und Pneumolysin (PLY). Experimentell getestet wurde Heparin als Natriumsalz (CAS Nr. 9041-08-1) (Polymer, isoliert aus Schweine-Mukosa, Molekulargewicht 2-25 kDa) sowie der Einsatz anderer Heparine (nieder- oder hochmolekular), wie Figur 8 zeigt. Bestätigt wurde die vorteilhafte Eigenschaft von Heparin mit Enoxaparin-Natrium (Clexane®, niedermolekulares Heparin) und Medunasal (unfraktioniertes Heparin) im Hämolysintitertest.

Der Hämolysintitertest wird zur Bestimmung der hämolytischen Aktivität in einer biologischen Probe eingesetzt. Dazu wird die biologische Probe und die zu testende Substanz (z.B. LLO) in verschiedenen Konzentrationen á 50 µl in 96 well Mikrotiterplatte gegeben. Anschließend werden Schafserythrozyten in Phosphatpuffer zu einer 1% Erythrozytensuspension angesetzt. Diese Suspension wird zu der biologischen Probe und zu der zu testenden Substanz á 50 µl in die 96 well Mikrotiterplatte gegeben. Jede Reihe enthält eine Erythrozytensuspension von 0,5%, wobei eine Kontrollreihe zur Verfügung steht. Die Platte wird 1h bei 37°C mit dem zu testenden Toxin inkubiert. Anschließend wird die gesamte Titerplatte zentrifugiert. Wenn Hämolyse eintritt, entsteht kein Hämoglobin-Pellet am Boden der Kavität und der Überstand verfärbt sich rötlich.

Heparin steigert die β-hämolytische Wirkung von LLO-produzierenden *L. monocytogenes*-Stämmen. Durch die Steigerung der hämolytischen Aktivität sinkt die Nachweisgrenze des LLO und somit auch die der Bakterien auf Blutagar-Platten. Damit kann diese Technik in der Diagnostik zur Detektion von CDC-Toxin produzierenden Mikroorganismen wie z.B. *L. monocytogenes* in biologischen Proben von Menschen oder Tieren, aber auch in Lebensmitteln oder in Lebensmittelproben verwendet werden.

Die Steigerung der hämolytischen Eigenschaften von LLO durch Heparin führt zur Bildung von besser sichtbaren Zonen mit β-Hämolyse auf Blutagar-Platten. Zusätzlich kann die Steigerung der β-Hämolyse auch mittels eines Cytotoxizitätstests, bei dem die Zelllyse gemessen wird, ermittelt werden.

Damit können in der Diagnostik die biologischen Proben schnell und einfach auf das Vorhandensein von LLO-positiven *L. monocytogenes-*Stämmen getestet werden. Damit ist diese Erfindung für die Diagnostik von entscheidender Bedeutung. Die Erhöhung der β-hämolytischen Wirkung von LLO durch Heparin setzt die Nachweisgrenze des LLO's herab. LLO wird von allen virulenten *L. monocytogenes*-Stämmen produziert. Damit ist diese Diagnostik insbesondere auch für die Lebensmittelindustrie, bei der Herstellung, Verarbeitung und Lagerung von Lebensmitteln von Bedeutung.

### Ausführungsbeispiele

Eigene wissenschaftliche Experimente belegen die vorteilhafte Wirkung des Heparins und damit versetzten Mediums und die dadurch verbesserten Verfahren Hämolysintitertest sowie dem Cytotoxizitätstest zur Detektion von CDC-Toxin produzierenden Mikroorganismen in einer biologischen Probe. Dargestellt ist die Wirkung von Heparin als Mittel zur Steigerung der β-Hämolyse von CDC-Toxin produzierenden Mikroorganismen beispielhaft für die Detektion von *Listeria monocytogenes* und *Streptococcus pneumoniae* gezeigt.

Die Ergebnisse sind aber ebenfalls anzuwenden auf alle anderen in einer biologischen Probe möglicherweise enthaltenden CDC-Toxin produzierende Mikroorganismen insbesondere pathogene Bakterien wie z.B. *Clostridium perfringens, Bacillus cereus, Bacillus anthracis, Streptococcus pyogenes* und *Clostridium tetani.*

Sie ist ebenfalls anzuwenden auf Bakterien, die Proteine mit einer mindestens 40%igen Aminosäuresequenzidentität mit CDC-Toxinen gemäß Seq.ID1 aufweisen.

### 1. Herstellung des verbesserten Differenzierungsnährbodens

### In einer Ausführung am Beispiel Blutagar:

30 g Standard Agar z.B. Columbia Agar (Oxoid) wird in 750 ml deionisiertem Wasser gelöst und bei 121°C 15 Minuten autoklaviert. Die Agar-Lösung wird auf 50°C abgekühlt. Dazu wird 37,5 ml defibriniertes Schafsblut (Oxoid) und 0,1 mg/ml Heparin, gelöst in phosphatgepufferter Salzlösung (kurz *PBS* von englisch phosphate buffered saline) beigefügt. Als Kontrolle wird statt Heparin nur PBS in die Agar-Blut-Lösung gegeben. Je 25,5 ml der Lösung wird in die entsprechenden noch leeren Plastikplatten gegossen. Nach dem Festwerden kann dieser verbesserte Differenzierungsnährboden enthaltend Heparin über eine Zeit von mind. 1 Monat bei 4°C gelagert werden. Nach dem Auftragen einer biologischen Probe z.B. einer Blut-, Serum- oder Stuhlprobe, einer Probe aus einem Gewebe oder Organ, ein Bioptat, ein Kosmetikum, aber auch eine Lebensmittelprobe, eine Rückstellprobe enthaltend ein verarbeitetes Lebensmittel oder eine Probe aus der Umwelt, wie Boden- oder Wasserproben, weist eine β-Hämolyse auf CDC-Toxin produzierende Mikroorganismen hin. Erfindungsgemäß ist es möglich, auch andere Arten von Agar einzusetzen, um die Steigerung der β-Hämolyse zu erzielen. Weiterhin kann statt Schafsblut auch Blut eines anderen Wirbeltieres z.B. Rind, Pferd oder Schwein zur Herstellung des Blutagars verwendet werden. Erfindungsgemäß ist es ebenfalls möglich, auch andere Heparine einzusetzen, um die gewünschte Wirkung als Mittel zur Steigerung der β-Hämolyse von CDC-Toxin produzierenden Mikroorganismen zu erzielen. Bei der Herstellung der Platten wird Heparin entweder der flüssigen Blutagar-Lösung zugefügt oder auf die feste Oberfläche einem fertigen bereits verfestigten Blutagar aufgebracht.

Die Konzentration des Heparins bei der eine Steigerung der β-Hämolyse von CDC-Toxin produzierenden Mikroorganismen sichtbar wird, liegt im Bereich von 1 pg/ml bis 5 mg/ml oder 0,00001 UI bis 1000 UI. Somit sind die CDC-Toxin produzierenden Mikroorganismen gut zu detektieren.

### 2. Detektion von CDC-Toxin produzierenden Mikroorganismen mittels des verbesserten Differenzierungsnährbodens am Beispiel von L. monocytogenes

Verschiedene Serotypen von *L. monocytogenes, L. monocytogenes* EGDeΔ*prfA* (eine Mutante, der der LLO-Transkriptionsfaktor fehlt), *L. monocytogenes* EG-DeΔ*hly* (eine Mutante, der das LLO fehlt), *L. innocua* (nicht pathogen), *L. welshimeri* (nicht pathogen) und LLO-produzierende *L. innocua* werden auf dem verbesserten Differenzierungsnährboden enthaltend Heparin mittels fraktioniertem Verdünnungsausstrich aufgebracht. Nur die pathogenen *L. monocytogenes* und LLO-produzierende *L. innocua,* jedoch nicht *L. monocytogenes* EGDeΔ*prfA, L. monocytogenes* EGDeΔ*hly, L. innocua* und *L. weshimeri* zeigen eine deutliche β-Hämolyse. Als Kontrolle wurde ein Differenzierungsnährboden nur mit PBS ohne Heparin verwendet.

Als Differenzierungsnährboden wird hier beispielhaft Blutagar verwendet. Auf Platten ohne Heparin ist die β-Hämolyse bei pathogenen *L. monocytogenes* nicht zu sehen bzw. auch nach sehr langer Inkubationszeit sehr viel weniger deutlich ausgeprägt als bei Blutagar mit Heparin.

Die Steigerung der β-Hämolyse auf diesem Differenzierungsnährboden ist auch bei anderen Bakterien, die CDC-Toxine produzieren, zu beobachten. Beispielsweise wird dies für *Streptococcus pneumoniae* gezeigt. Dabei wurde *L. innocua,* welches das CDC-Toxin Pneumolysin (PLY) von S. *pneumoniae* produziert, auf dem verbesserten Differenzierungsnährboden z.B. Blutagar enthaltend Heparin mittels fraktioniertem Verdünnungsausstrich aufgebracht. Bei Blutagar mit Heparin zeigen PLY-positive *L. innocua* eine deutliche β-Hämolyse und sind somit gut zu detektieren.

### 3. Detektion von CDC-Toxin produzierenden Mikroorganismen mittels verbesserten Hämolysintitertest mit Heparin am Beispiel von LLO

Die Detektion von CDC-Toxin produzierenden Mikroorganismen kann neben dem Heparin enthaltenden Differentialnährboden auch mittels eines Hämolysintitertests mit Heparinlösung in einer Konzentration von 1 pg/ml bis 5 mg/ml oder 0,00001 UI bis 1000 UI erfolgen.

Damit kann die hämolytische Aktivität einer biologischen Probe besser bestimmt werden und auf das Vorhandensein von CDC-Toxin produzierenden Mikroorganismen geschlossen werden. Dazu nutzt man aus einer biologischen Probe den Überstand oder eine Flüssigkeit, in der CDC-Toxin produzierende Mikroorganismen vermutet werden.

### In einer Ausführung:

3 ml Schafsblut (ACILA AG) werden mit 1x PBS gemischt und abzentrifugiert (2500 rpm, 3 min, 10°C). Der Überstand wird verworfen und das Blut so oft gewaschen, bis der Überstand klar ist. Der Überstand wird komplett abgenommen und 100 µl der Erythrozyten mit 9,9 ml 1x PBS gemischt.

Für den Test werden 50 µl 1x PBS versetzt mit 1:500 β-Mercaptoethanol in 96-well V-Platte (z.B. 96 Well Polystyrol Microplatten von Greiner Bio-One) vorgelegt. Das "V" steht für den konisch zulaufenden Näpfchenboden. 100 µl der LLO-Lösung (hier wird aufgereinigtes LLO verwendet) wird in das erste (leere) well der V-Platte pipettiert. Es wird eine 1:2-Verdünnungsreihe erstellt, indem man aus dem ersten well 50 µl der Lösung heraus nimmt, in das zweite well pipettiert und mischt. Erneut werden 50 µl der Lösung heraus genommen und in das dritte well gegeben, usw.

Als negative Kontrolle wird 50 µl 1x PBS versetzt mit 1:500 β-Mercaptoethanol mit bzw. ohne Heparin verwendet und als positive Kontrolle 50 µl ddH₂O. In alle wells werden 50 µl von der verdünnten Erythrozytenlösung und 20 µl der Heparinlösung mit einer Endkonzentration von 1 pg/ml bis 5 mg/ml oder 0,00001 UI bis 1000 UI gegeben. Die Platte wird eine Stunde bei 37°C inkubiert. Die Platte wird abzentrifugiert (1000 rpm, 2 min, Raumtemperatur), 50 µl des Überstandes werden in eine 96-well Rundboden-Platte überführt und die Absorption bei 405 nm gemessen. Anstelle der LLO-Lösung wird bei der Diagnostik einer biologischen Probe 100 µl einer flüssigen oder verflüssigten Probe oder eines Überstandes einer Bakterienkultur verwendet.

Alternativ wird statt Schafsblut das Blut eines anderen Wirbeltieres z.B. Rind, Pferd oder Schwein verwendet.

Erfindungsgemäß ist es ebenfalls möglich auch andere Formen des Heparins (nieder- oder hochmolekular oder unfraktioniertes Heparin) einzusetzen, um die gewünschte Wirkung als Mittel zur Steigerung der β-Hämolyse von CDC-Toxin produzierenden Mikroorganismen zu erzielen. Weiterhin können Heparin-Oligosaccharide oder modifizierte Heparine (z.B. Gallensäure-acyliertes Heparinderivat) verwendet werden, die nach einem dem Fachmann bekannten Verfahren synthetisch hergestellt wurden.

Die Heparinlösung wird stets in einer Endkonzentration von 1 pg/ml bis 5 mg/ml oder 0,00001 UI bis 1000 UI verwendet. Alternativ erfolgt eine Inkubation der CDC-Toxin-Blut-Heparin-Lösung. In allen Alternativen ist stets eine Steigerung der β-Hämolyse von CDC-Toxin produzierenden Mikroorganismen festzustellen und damit eine verbesserte Detektion auf diese Mikroorganismen in einer biologischen Probe möglich.

### 4. Detektion von CDC-Toxin produzierenden Mikroorganismen mittels verbesserter Cytotoxizitätsmessung mit Heparin

Zusätzlich kann die Detektion von CDC-Toxin produzierenden Mikroorganismen mittels Cytotoxitätstest mit Heparin bestimmt werden. Hierbei werden adhärente (anhaftende) oder nicht adhärente (nicht anhaftende) eukaryotische kernhaltige oder kernlose Zellen in Platten kultiviert. Anschließend werden die Zellen mit CDC-Toxin enthaltenden Lösungen inkubiert und das Überleben der Zellen bestimmt. Bei der Diagnostik wird anstelle der CDC-Toxin enthaltenden Lösungen eine biologische Probe oder ein Überstand einer Bakterienkultur zugesetzt.

### In einer Ausführung:

HeLa Zellen werden über Nacht in 24-well-Platten in 500 µl 10%-FKS (FKS = Fötales Kälberserum) haltigem Medium kultiviert, so dass sie eine 90-100% Konfluenz erreichen. Kurz vor der Behandlung mit aufgereinigter LLO-Lösung werden die Zellen 5x mit Hanks Salt Solution gewaschen, um das im Medium enthaltende Serum (enthält Cholesterin, welches CDC-Toxine inhibiert) zu entfernen. Die Zellen werden mit Heparin-enthaltendem Medium (ohne FKS) versetzt.

Heparin wird dabei in einer Konzentration von 1 pg/ml bis 5 mg/ml oder 0,00001 UI bis 1000 UI verwendet.

Die LLO-haltige Lösung wird sofort zu den Zellen gegeben und bei 37°C in 5%iger CO₂ Umgebung eine Stunde inkubiert. Anschließend wird das Medium abgenommen und durch 500 µl MMT-haltige Lösung (1 ml MTT [5 mg/ml in PBS] mit 9 ml 10% FKS-haltigem Medium) ersetzt. MTT ist 3-(4,5-Dimethylthiazol-2-yl)-diphenyltetrazoliumbromid. Die Zellen werden zwei Stunden bei 37°C in 5%iger CO₂ Umgebung inkubiert. Anschließend wird das Medium abgesaugt und 500 µl Stopplösung (5% Ameisensäure in Isopropanol) pro well pipettiert. Die Platte wird auf einem Plattenschüttler gevortext und die Absorption bei 562 nm bestimmt.

Beispielhaft werden hier alle Experimente zur Anwendung des Heparin-haltigen Differentialnährbodens, des Hämolysintitertests mit Heparin und des Cytotoxizitätstests mit Heparin am CDC-produzierenden Mikroorganismus *L. monocytogenes* gezeigt. Die Ergebnisse zeigen, dass Heparin in einer Konzentration von 1 pg/ml bis 5 mg/ml oder 0,00001 UI bis 1000 UI ein geeignetes Mittel zur Steigerung der β-Hämolyse ist. Die Ergebnisse zeigen auch, dass mit Heparin die bereits bekannten Verfahren zur Detektion von CDC-produzierenden Mikroorganismen in biologischen Proben erheblich verbessert werden, so dass eine zuverlässigere, sichere und wesentlich schnellere Diagnostik auf CDC-produzierende Mikroorganismen in biologischen Proben zur Verfügung steht.

### Aminosäuresequenzen

### Abbildungen

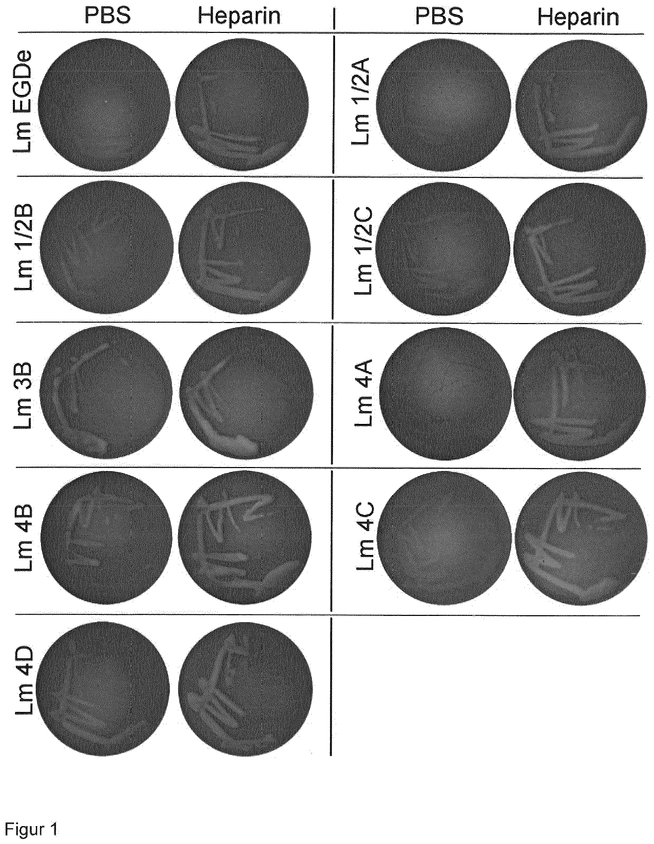

**Figur 1** zeigt die Auswertung der Anwendung des verbesserten Differenzierungsnährbodens mit Heparin anhand von Blutagar-Platten enthaltend 0,1 mg/ml Heparin (oder als Kontrolle PBS) mit verschiedenen Serotypen von *L. monocytogenes.* Die Serotypen sind mit Lm bezeichnet und wurden mittels fraktioniertem Verdünnungsausstrich auf die Platte aufgebracht und über Nacht bei 37°C inkubiert.

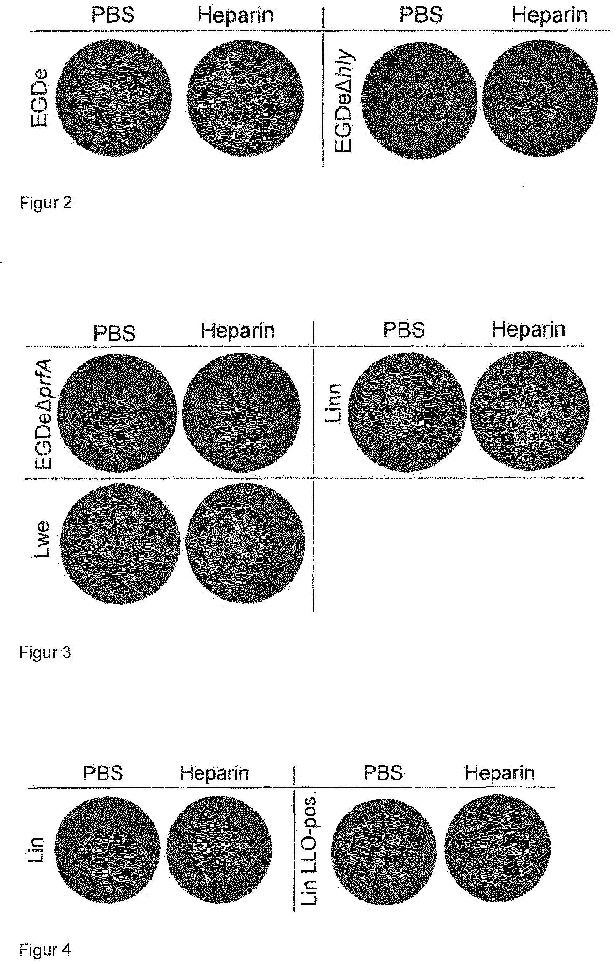

**Figur 2** zeigt die Auswertung der Anwendung des verbesserten Differenzierungsnährbodens mit Heparin anhand von Blutagar-Platten enthaltend 0,1 mg/ml Heparin (oder als Kontrolle PBS) mit *L. monocytogenes* EGDe (als EGDe bezeichnet) und *L. monocytogenes* EGDeΔ*hly* (eine Mutante, der das LLO fehlt; als EGDeΔ*hly* bezeichnet). Die Bakterien wurden mittels fraktioniertem Verdünnungsausstrich auf die Platte aufgebracht und zwei Tage bei 37°C inkubiert.
**Figur 3** zeigt die Auswertung der Anwendung des verbesserten Differenzierungsnährbodens mit Heparin anhand von Blutagar-Platten enthaltend 0,1 mg/ml Heparin (oder als Kontrolle PBS) mit *L. monocytogenes* EGDeΔ*prfA* (eine Mutante, der der LLO-Transkriptionsfaktor fehlt; als EGDeΔ*prfA* bezeichnet), *L. innocua* (nicht pathogen; als Linn bezeichnet) und *L. welshimeri* (nicht pathogen; als Lwe bezeichnet). Die Bakterien wurden mittels fraktioniertem Verdünnungsausstrich auf die Platte aufgebracht und über Nacht bei 37°C inkubiert.
**Figur 4** zeigt die Auswertung der Anwendung des verbesserten Differenzierungsnährbodens mit Heparin anhand von Blutagar-Platten enthaltend 0,1 mg/ml Heparin (oder als Kontrolle PBS) mit *L. innocua* (nicht pathogen; als Lin bezeichnet) und LLO-produzierenden *L. innocua* (als Lin LLO-pos. bezeichnet). Die Bakterien wurden mittels fraktioniertem Verdünnungsausstrich auf die Platte aufgebracht und zwei Tage bei 37°C inkubiert.

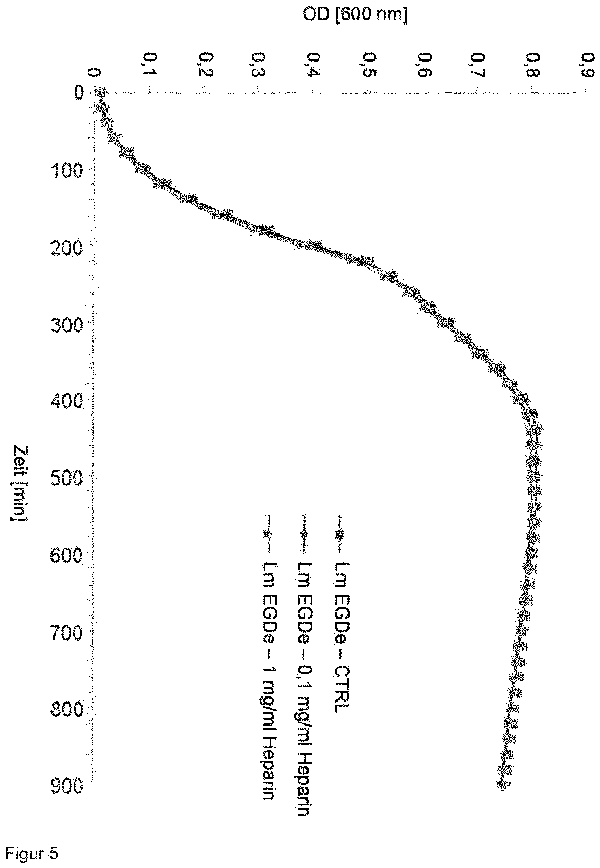

**Figur 5** zeigt die Wachstumskurven von *L. monocytogenes* (Lm) EGDe ohne Heparinzugabe (CTRL) und mit 0,1 mg/ml Heparin oder 1 mg/ml Heparin.
   Die verwendete Heparinkonzentration von 0,1 mg/ml oder eine Konzentration von 1 mg/ml hat keinen Einfluss auf das Wachstum von *L. monocytogenes* EGDe. Dabei wurde eine Übernachtkultur von *L. monocytogenes* EGDe 1:50 in Brain-Heart-Infusion broth verdünnt. 200 µl der verdünnten Bakterienkultur wurde in Gegenwart von Heparin in einer 96-well Platte bei 37°C im InfiniteM200 Plattenlesegerät inkubiert. Alle 20 Minuten wurde die Platte für 200 Sekunden geschüttelt und die Absorption bei 600 nm gemessen.

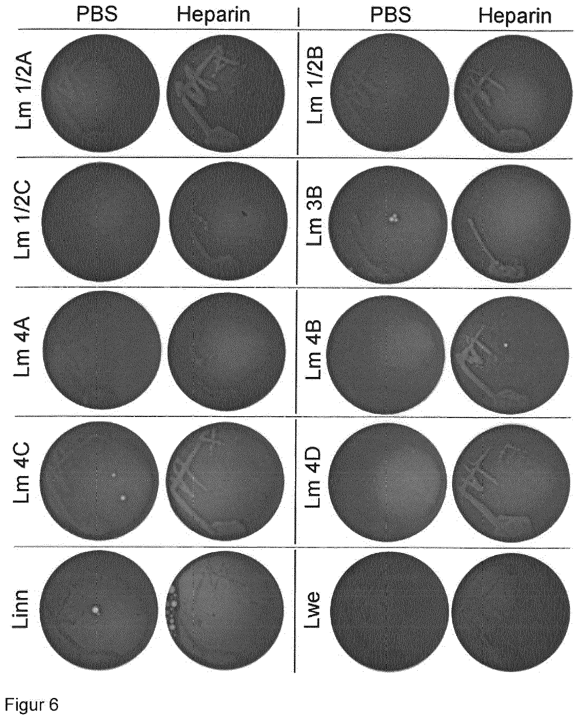

**Figur 6** zeigt die Stabilität des verbesserten Differenzierungsnährbodens mit Heaprin anhand von Blutagar-Platten enthaltend 0,1 mg/ml Heparin. Um die Stabilität des Heparin (0,1 mg/ml) enthaltenden Differenzierungsnährbodens zu bestimmen, wurden die Platten bei 4°C aufbewahrt. Nach einem Monat wurden verschiedene Serotypen von *L. monocytogenes* (als Lm bezeichnet), *L. innocua* (als Linn bezeichnet) und *L. whelshimeri* (als Lwe bezeichnet) mittels fraktioniertem Verdünnungsausstrich auf die Platte aufgebracht und über Nacht bei 37°C inkubiert. Figur 6 zeigt deutlich, dass auf den 1 Monat alten Platten die Steigerung der β-Hämolyse immer noch gegeben ist. Daraus wird abgeleitet, dass die Platten mind. einen Monat haltbar sind.

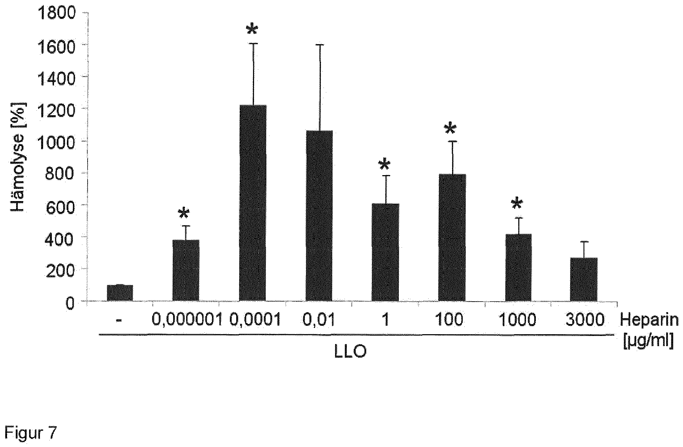

**Figur 7** zeigt die Steigerung der β-Hämolyse von LLO durch Heparin im verbesserten Hämolysintitertest. Im Hämolysintitertest werden Schafserythrozyten mit aufgereinigtem LLO in Gegenwart von Heparin (oder als Kontrolle PBS) in einer V-förmigen 96-well Platte eine Stunde bei 37°C inkubiert. Die Platte wird abzentrifugiert und die Absorption bei 405 nm gemessen.
   Figur 7 zeigt die deutliche Steigerung der β-Hämolyse von LLO. Die Sterne zeigen die Signifikanz (*p<0,05) von Heparin behandelten LLO zu LLO ohne Heparin.
**Figur 8** zeigt die Steigerung der β-Hämolyse von LLO durch niedermolekulares Heparin Enoxaparin-Natrium und unfraktioniertes Heparin Medunasal ermittelt im verbesserten Hämolysintitertest. Sie zeigt, dass sowohl niedermolekulares als auch hochmolekulares Heparin die β-Hämolyse steigert und für die Detektion der CDC-Toxin produzierende Mikroorganismen eingesetzt werden kann.
   Dabei wird Enoxaparin-Natrium (als Clexane® von Sanofi benannt, ein niedermolekulares Heparin) und Medunasal (PZN 6093089 von Sintetica, ein unfraktioniertes Heparin) verdünnt und mit aufgereinigtem LLO und Schafsblut eine Stunde bei 37°C in einer V-förmigen Platte inkubiert. Als Kontrolle dient PBS mit und ohne Enoxaparin-Natrium sowie Medunasal. Die Platte wird abzentrifugiert und die Absorption bei 405 nm gemessen.
   Dargestellt ist die deutliche Steigerung der β-Hämolyse von LLO durch Enoxaparin-Natrium und Medunasal. Die Sterne zeigen die Signifikanz (*p<0,05) von Heparin behandelten LLO zu LLO ohne Heparin.

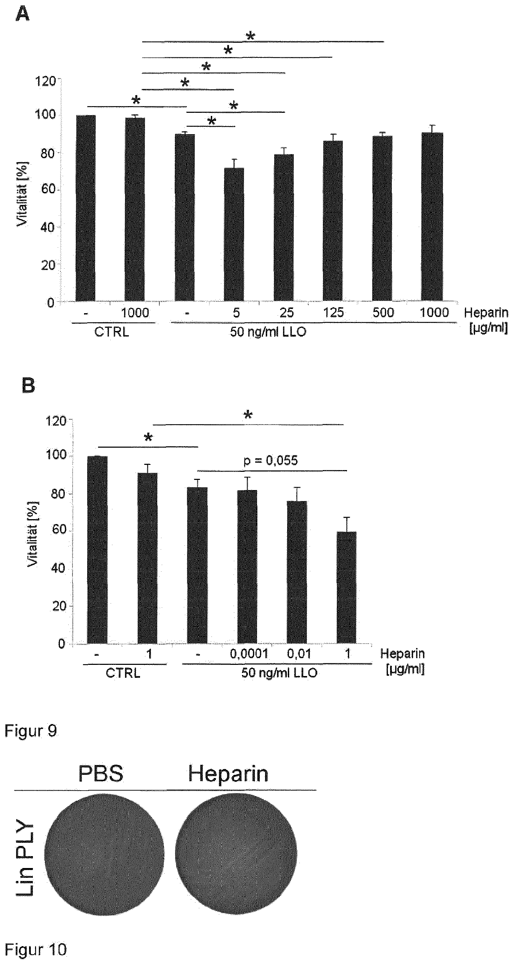

**Figur 9** zeigt die konzentrationsabhängige Steigerung der β-Hämolyse von LLO durch Heparin ermittelt im verbesserten Cytotoxitätstest. Hierbei werden HeLa Zellen mit aufgereinigtem LLO in Gegenwart von Heparin (im fötalem Kälberserum FKS-freien Medium) in einer 24-well-Platte eine Stunde bei 37°C und 5% CO₂ inkubiert.
   Anschließend wird das Medium abgenommen und die Zellen mit MTT-haltigem Medium (10% FKS) 2h bei 37°C und 5% CO₂ inkubiert. Nach der Inkubationsphase wird die Diphenyltetrazoliumbromid Thiazylblau (MTT)-Lösung abgenommen, die Zellen in der Stopplösung lysiert und die Absorption bei 562 nm gemessen. Die Sterne zeigen die Signifikanz (*p<0,05).
**Figur 10** zeigt die Auswertung der Anwendung des verbesserten Differenzierungsnährbodens anhand von Blutagar-Platten enthaltend 0,1 mg/ml Heparin oder als Kontrolle PBS mit PLY-produzierenden *L. innocua* (bezeichnet als Lin PLY). PLY ist das CDC-Toxin von S. *pneumoniae.* Die Bakterien wurden mittels fraktioniertem Verdünnungsausstrich auf die Blutagar-Platte aufgebracht und über Nacht bei 37°C inkubiert. Es ist deutlich zu erkennen, dass durch Heparin die β-Hämolyse von PLY gesteigert wird.
   Dies zeigt, dass die Erfindung auch auf andere CDC-produzierende Mikroorganismen anzuwenden ist.

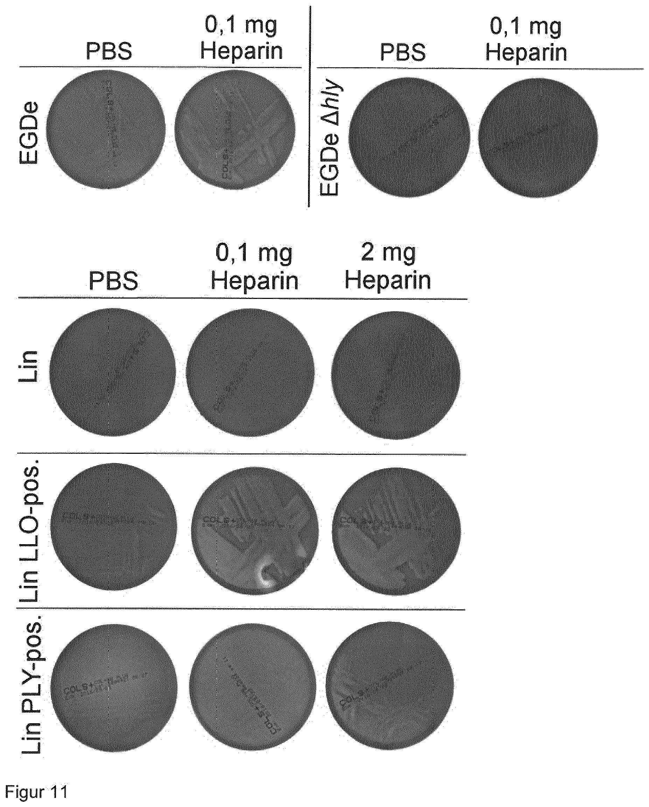

**Figur 11** zeigt die Auswertung der Blutagar-Platten, wenn Heparin direkt auf die Oberfläche der fertigen und verfestigten Blutagar-Platten aufgetragen wurde. Für diese Experimente wurde 0,1 mg oder 2 mg Heparin eingesetzt. Die Bakterien *L*. *monocytogenes* EGDe (als EGDe bezeichnet), *L. monocytogenes* Δ*hly* (als EG-DeΔ*hly* bezeichnet), *L. innocua* (als Lin bezeichnet), LLO-produzierende *L. innocua* (als Lin LLO-pos. bezeichnet) und PLY-produzierende *L. innocua* (als Lin PLY-pos. bezeichnet) wurden mittels fraktioniertem Verdünnungsausstrich aufgebracht und über Nacht bei 37°C inkubiert.

## Patentansprüche

1. Verwendung eines Differentialnährbodens umfassend Heparin zur Diagnostik von CDC-Toxin produzierenden Bakterien wobei eine biologische Probe auf dem Differentialnährboden ausgebracht, kultiviert und auf das Vorhandensein von β-Hämolyse ausgewertet wird.

2. Verwendung gemäß Anspruch 1 **dadurch gekennzeichnet dass** Heparin fraktioniertes/niedermolekulares und/oder unfraktioniertes/hochmolekulares Heparin, synthetische Heparin-Oligosaccharide oder Heparansulfate umfasst.

3. Verwendung gemäß der Ansprüche 1 oder 2 **dadurch gekennzeichnet dass** Heparin in einer Konzentration von 1 pg/ml - 5 mg/ml oder 0,00001 UI - 1000 UI eingesetzt wird.

4. Verwendung gemäß einem der vorangehenden Ansprüche **dadurch gekennzeichnet dass** der Differentialnährboden ein Blutagar ist.

5. Verwendung gemäß einem der vorangehenden Ansprüche 1-4 **dadurch gekennzeichnet dass** das Vorhandensein von β-Hämolyse im Hämolysintitertest ausgewertet wird.

6. Verwendung gemäß einem der vorangehenden Ansprüche 1-4 **dadurch gekennzeichnet dass** das Vorhandensein von β-Hämolyse im Cytotoxizitätstest ausgewertet wird.

7. Verwendung gemäß einem der vorangehenden Ansprüche **dadurch gekennzeichnet dass** die CDC-Toxin produzierenden Bakterien ausgewählt sind aus der Gruppe *Arcanobacterium pyogenes, Bacillus anthracis, Bacillus cereus, Bacillus sphaericus, Bacillus thuringiensis, Brevibacillus laterosporus, Brevibacillus brevis, Clostridium bifermentans, Clostridium botulinum, Clostridium butyricum, Clostridium chauvoei, Clostridium histolyticum, Clostridium novyi, Clostridium perfringens, Clostridium septicum, Clostridium sordelli, Clostridium tetani, Gardnerella vaginalis, Listeria ivanovii, Listeria monocytogenes, Listeria seeligeri, Paenibacillus alvei, Streptococcus canis, Streptococcus dysgalactiae, Streptococcus intermedius, Streptococcus mitis, Streptococcus pneumoniae, Streptococcus pyogenes, Streptococcus suis, Streptococcus pseudopnemoniae.*

8. Verwendung gemäß einem der vorangehenden Ansprüche **dadurch gekennzeichnet dass** die CDC-Toxin produzierenden Bakterien eine mindestens 40%ige Aminosäuresequenzidentität zu Seq.ID1 aufweisen.

9. Verwendung gemäß einem der vorangehenden Ansprüche **dadurch gekennzeichnet dass** die biologische Probe eine Blut-, Serum- oder Stuhlprobe, eine Probe aus einem Gewebe oder Organ, ein Bioptat, ein Kosmetikum, eine Lebensmittelprobe, eine Rückstellprobe enthaltend ein verarbeitetes Lebensmittel oder eine Probe aus der Umwelt, wie Boden- oder Wasserprobe ist.

## Claims

1. Use of a differential culture medium comprising heparin for the diagnosis of CDC toxin-producing bacteria, wherein a biological sample is applied to the differential culture medium, cultivated and evaluated for the presence of β-haemolysis.

2. Use according to claim 1 **characterised in that** heparin comprises fractionated/low molecular weight and/or unfractionated/high molecular weight heparin, synthetic heparin oligosaccharides or heparan sulphates.

3. Use according to claims 1 or 2 **characterized in that** heparin is used in a concentration of 1 pg/ml - 5 mg/ml or 0.00001 UI - 1000 UI.

4. Use according to one of the preceding claims **characterized in that** the differential culture medium is a blood agar.

5. Use according to any of claims 1-4 above **characterized by** evaluating the presence of β hemolysis in the hemolysin titre test.

6. Use according to any of claims 1-4 above **characterized by** evaluating the presence of β hemolysis in the cytotoxicity test.

7. Use according to one of the preceding claims **characterized in that** the CDC toxin producing bacteria are selected from the group Arcanobacterium pyogenes, Bacillus anthracis, Bacillus cereus, Bacillus sphaericus, Bacillus thuringiensis, Brevibacillus laterosporus, Breviba-cillus brevis, Clostridium bifermentans, Clostridium botulinum, Clostridium butyricum, Clostridium chauvoei, Clostridium histolyticum Clostridium novyi, Clostridium perfringens, Clostridium septicum, Clostridium sordelli, Clostridium tetani, Gardnerella vaginalis, Listeria ivanovii, Listeria monocytogenes, Listeria seeligeri, Paenibacillus alvei, Streptococcus canis, Streptococcus dysgalactiae, Streptococcus intermedius, Streptococcus mitis, Streptococcus pneumoniae, Streptococcus pyogenes, Streptococcus suis, Streptococcus pseudopnemoniae.

8. Use according to any of the foregoing claims **characterized in that** the CDC toxin producing bacteria have at least 40% amino acid sequence identity to SEQ.ID1.

9. Use according to one of the preceding claims **characterized in that** the biological sample is a blood, serum or stool sample, a tissue or organ sample, a bioptate, a cosmetic, a food sample, a retain sample containing a processed food or an environmental sample, such as soil or water sample.

## Revendications

1. Utilisation d'un milieu de culture différentielle comprenant de l'héparine pour le diagnostic des bactéries productrices de toxines CDC, dans laquelle un échantillon biologique est appliqué sur le milieu de culture différentielle, cultivé et évalué pour la présence de l'hémolyse β.

2. Utilisation selon la revendication 1, **caractérisée en ce que** l'héparine comprend de l'héparine fractionnée/à bas poids moléculaire et/ou non fractionnée/à haut poids moléculaire, des oligosaccharides d'héparine synthétique ou des sulfates d'héparane.

3. Utilisation selon les revendications 1 ou 2 **caractérisée en ce que** l'héparine est utilisée à une concentration de 1 pg/ml - 5 mg/ml ou 0,00001 UI - 1000 UI.

4. Utilisation selon l'une des revendications précédentes **caractérisée en ce que** le milieu de culture différentiel est une gélose au sang.

5. Utilisation selon l'une des revendications 1-4 ci-dessus, **caractérisée par** l'évaluation de la présence d'une hémolyse β dans le test du titre d'hémolysine.

6. Utilisation selon l'une des revendications 1 à 4 ci-dessus, **caractérisée par** l'évaluation de la présence d'une hémolyse β dans le test de cytotoxicité.

7. Utilisation selon l'une des revendications précédentes **caractérisée en ce que** les bactéries productrices de toxines du CDC sont sélectionnées dans le groupe Arcanobacterium pyogenes, Bacillus anthracis, Bacillus cereus, Bacillus sphaericus, Bacillus thuringiensis, Brevibacillus laterosporus, Brevibacillus brevis, Clostridium bifermentans, Clostridium botulinum, Clostridium butyricum, Clostridium chauvoei, Clostridium histolyticum Clostridium novyi, Clostridium perfringens, Clostridium septicum, Clostridium sordelli, Clostridium tetani, Gardnerella vaginalis, Listeria ivanovii, Listeria monocytogenes, Listeria seeligeri, Paenibacillus alvei, Streptococcus canis, Streptococcus dysgalactiae, Streptococcus intermedius, Streptococcus mitis, Streptococcus pneumoniae, Streptococcus pyogenes, Streptococcus suis, Streptococcus pseudopnemoniae.

8. Utilisation selon l'une des revendications précédentes **caractérisée en ce que** les bactéries productrices de toxines du CDC ont au moins 40% d'identité de séquence d'acides aminés avec la SEQ ID1.

9. Utilisation selon l'une des revendications précédentes **caractérisée en ce que** l'échantillon biologique est un échantillon de sang, de sérum ou de selles, un échantillon provenant d'un tissu ou d'un organe, un bioptate, un cosmétique, un échantillon alimentaire, un échantillon conservé contenant un aliment transformé ou un échantillon provenant de l'environnement, tel qu'un échantillon de sol ou d'eau.


### SEQUENCE LISTING

<110> Justus-Liebig-Universität
<120> Erfindung betreffend die Steigerung der -Hämolyse von CDC-Toxin
   produzierenden Mikroorganismen
<130> EP
<160> 1
<170> BiSSAP 1.3.5
<210> 1
   <211> 529
   <212> PRT
   <213> Listeria monocytogenes
<400> 1
   Met Lys Lys Ile Met Leu Val Phe Ile Thr Leu Ile Leu Val Ser Leu
   1               5                   10                  15
   Pro Ile Ala Gln Gln Thr Glu Ala Lys Asp Ala Ser Ala Phe Asn Lys
               20                  25                  30
   Glu Asn Ser Ile Ser Ser Met Ala Pro Pro Ala Ser Pro Pro Ala Ser
           35                  40                  45
   Pro Lys Thr Pro Ile Glu Lys Lys His Ala Asp Glu Ile Asp Lys Tyr
       50                  55                  60
   Ile Gln Gly Leu Asp Tyr Asn Lys Asn Asn Val Leu Val Tyr His Gly
   65                  70                  75                  80
   Asp Ala Val Thr Asn Val Pro Pro Arg Lys Gly Tyr Lys Asp Gly Asn
                   85                  90                  95
   Glu Tyr Ile Val Val Glu Lys Lys Lys Lys Ser Ile Asn Gln Asn Asn
               100                 105                 110
   Ala Asp Ile Gln Val Val Asn Ala Ile Ser Ser Leu Thr Tyr Pro Gly
           115                 120                 125
   Ala Leu Val Lys Ala Asn Ser Glu Leu Val Glu Asn Gln Pro Asp Val
       130                 135                 140
   Leu Pro Val Lys Arg Asp Ser Leu Thr Leu Ser Ile Asp Leu Pro Gly
   145                 150                 155                 160
   Met Thr Asn Gln Asp Asn Lys Ile Val Val Lys Asn Ala Thr Lys Ser
                   165                 170                 175
   Asn Val Asn Asn Ala Val Asn Thr Leu Val Glu Arg Trp Asn Glu Lys
               180                 185                 190
   Tyr Ala Gln Ala Tyr Pro Asn Val Ser Ala Lys Ile Asp Tyr Asp Asp
           195                 200                 205
   Glu Met Ala Tyr Ser Glu Ser Gln Leu Ile Ala Lys Phe Gly Thr Ala
       210                 215                 220
   Phe Lys Ala Val Asn Asn Ser Leu Asn Val Asn Phe Gly Ala Ile Ser
   225                 230                 235                 240
   Glu Gly Lys Met Gln Glu Glu Val Ile Ser Phe Lys Gln Ile Tyr Tyr
                   245                 250                 255
   Asn Val Asn Val Asn Glu Pro Thr Arg Pro Ser Arg Phe Phe Gly Lys
               260                 265                 270
   Ala Val Thr Lys Glu Gln Leu Gln Ala Leu Gly Val Asn Ala Glu Asn
           275                 280                 285
   Pro Pro Ala Tyr Ile Ser Ser Val Ala Tyr Gly Arg Gln Val Tyr Leu
       290                 295                 300
   Lys Leu Ser Thr Asn Ser His Ser Thr Lys Val Lys Ala Ala Phe Asp
   305                 310                 315                 320
   Ala Ala Val Ser Gly Lys Ser Val Ser Gly Asp Val Glu Leu Thr Asn
                   325                 330                 335
   Ile Ile Lys Asn Ser Ser Phe Lys Ala Val Ile Tyr Gly Gly Ser Ala
               340                 345                 350
   Lys Asp Glu Val Gln Ile Ile Asp Gly Asn Leu Gly Asp Leu Arg Asp
           355                 360                 365
   Ile Leu Lys Lys Gly Ala Thr Phe Asn Arg Glu Thr Pro Gly Val Pro
       370                 375                 380
   Ile Ala Tyr Thr Thr Asn Phe Leu Lys Asp Asn Glu Leu Ala Val Ile
   385                 390                 395                 400
   Lys Asn Asn Ser Glu Tyr Ile Glu Thr Thr Ser Lys Ala Tyr Thr Asp
                   405                 410                 415
   Gly Lys Ile Asn Ile Asp His Ser Gly Gly Tyr Val Ala Gln Phe Asn
               420                 425                 430
   Ile Ser Trp Asp Glu Val Asn Tyr Asp Pro Glu Gly Asn Glu Ile Val
           435                 440                 445
   Gln His Lys Asn Trp Ser Glu Asn Asn Lys Ser Lys Leu Ala His Phe
       450                 455                 460
   Thr Ser Ser Ile Tyr Leu Pro Gly Asn Ala Arg Asn Ile Asn Val Tyr
   465                 470                 475                 480
   Ala Lys Glu Cys Thr Gly Leu Ala Trp Glu Trp Trp Arg Thr Val Ile
                   485                 490                 495
   Asp Asp Arg Asn Leu Pro Leu Val Lys Asn Arg Asn Ile Ser Ile Trp
               500                 505                 510
   Gly Thr Thr Leu Tyr Pro Lys Tyr Ser Asn Lys Val Asp Asn Pro Ile
           515                 520                 525
   Glu